# EUROPEAN PATENT APPLICATION

(11) **EP 1 254 670 A1**
(43) Date of publication of application: **06.11.2002**
(21) Application number: 01201633.3
(22) Date of filing: 03.05.2001
(51) Int. Cl.: A61L 27/24

(54) **Type II collagen matrices for use in cartilage engineering**

(71) Applicant: Stichting Katholieke Universiteit, 6525 EZ Nijmegen (NL)
(72) Inventor: Van Kuppevelt, Antonius Henricus Minardus Severus, 6536 BN Nijmegen (NL); Pieper, Jeroen Sebastiaan, 6532 WZ Nijmegen (NL); Van Moerkerk, Hermanus Theodorus Bernardus, 6578 BA Leuth (NL)
(74) Representative: Van Someren, Petronella F. H. M.

(57) **Abstract**

The present invention relates to a matrix for use in engineering or regeneration of cartilage, which matrix consists of a network of modified type II collagen. The network is crosslinked in case the modified type II collagen is type II atelocollagen. Alternatively, the modified type II collagen comprises type II collagen fibrils that have been treated with high cutting to obtain fibril parts.

## Description

The present invention relates to a type II collagen matrix, a type I and II composite matrix, methods for preparing them and the use thereof in cartilage engineering.

Articular cartilage has a limited potential to restore damage to the joint surfaces, which is related to the low mitotic activity of chondrocytes and the avascular nature of this tissue. Strategies like abrasion arthroplasty, excision, or the use of perichondreal or periosteal autografts, generally result in the formation of mechanically inferior fibro-cartilaginous tissue which may induce osteoarthritis. Tissue engineering of cartilage, in which biocompatible matrices are used, and in which chondrocytes can be cultured to prepare transplantable hyaline-like tissue, may provide a more suitable alternative.

Various insoluble biodegradable materials have been exploited as a carrier for chondrocytes. These include demineralized bone, hydroxyapatite composites, polylactic acid, polyglycolic acid, collagen, and hyaluronate. The physico-chemical characteristics of these materials were found to considerably influence the stability of the chondrocyte phenotype. Preservation of the chondrocytic phenotype is however essential for the generation and maintenance of hyaline-like cartilage. There is therefore a need for matrices which mimic articular cartilage biochemically, as these may add in preserving the differentiated state of the cells during culture.

The function of native articular cartilage as a load-bearing tissue is based on the unique structure of an organized extracellular network composed of mainly type II collagen and large aggregated proteoglycans (PGs), both in intimate contact. The major glycosaminoglycan (GAG) component of the monomeric PGs is chondroitin sulfate (CS), which constitutes for about 30 % of the cartilage dry weight. The inventors contemplated that matrices composed of type II collagen and chondroitin sulfate (CS), the major constituents of hyaline cartilage, may create an appropriate environment for the generation of cartilage-like tissue.

Since methods of preparing matrices of type I collagen are well-known, it would appear that type II collagen matrices could be made in an analogous manner. However, in the research that led to the invention it was found that these methods did not produce type II collagen matrices. An alternative method that produces a matrix of type II collagen is therefore desired.

An additional requirement of matrix material intended for use in the human and animal body is that it is pure. Furthermore, non-collagenous impurities, cellular remnants and residual PGs may elicit an immune response and should therefore also be absent.

Furthermore, a three-dimensional matrix is required, not only as a carrier for the transplantation of cells and for providing temporary mechanical support, but also for maintaining the characteristic round morphology of the chondrocytes.

In view of the above it is the object of the present invention to provide a method for the preparation of highly purified type II collagen, and the development of porous crosslinked type II collagen matrices.

This is achieved according to the invention by using a modified form of type II collagen as the starting material for the preparation of a matrix. More specifically the invention relates to a matrix for use in engineering or regeneration of cartilage, which matrix consists of a network of modified type II collagen. The network is optionally crosslinked depending on the kind of modified type II collagen.

The modification of type II collagen can be achieved in various ways, such as by enzyme treatment or by mechanical force.

In a first embodiment thereof the invention makes use of a type II collagen that has been treated with one or more enzymes, in particular pepsin. This treatment removes the terminal telopeptides of the collagen molecules, which are responsible for the crosslinking of the collagen molecules in a fibril. The treatment thus yields soluble molecules, which can be purified and again crosslinked to form the collagen type II matrix of the invention. It was found that pepsin treatment of type II collagen is necessary to obtain a pure product, and to fabricate matrices.

In accordance with this embodiment, the invention therefor provides a method for the preparation of an enzymatically modified type II collagen matrix as claimed, comprising the steps of:
a) providing a source of type II collagen;
b) enzymatically digesting the source of type II collagen to obtain enzymatically modified type II collagen;
c) preparing an non-crosslinked type II collagen matrix thereof; and
d) crosslinking the matrix thus obtained.

This embodiment is further described in Example 1, in which chondrocytes were cultured in crosslinked type II collagen matrices, with and without attached CS. Cell behavior in these matrices was compared to that in crosslinked type I collagen-CS matrices. Porous insoluble type I and type II collagen matrices were prepared by freeze-drying highly purified native type I collagen, and pepsin-treated type II collagen, respectively. Collagen crosslinking and CS attachment were performed using 1-ethyl-3-(3-dimethyl aminopropyl)carbodiimide.

Type I as well as type II matrices had a similar morphology and maintained their structural integrity during culture. After 7 days, chondrocytes were mainly located at the periphery of the matrices. In contrast to type I collagen, type II collagen supported the distribution of cells throughout the matrix. Bioactivity of chondrocytes was concluded from increased DNA and glycosaminoglycan contents. At the ultrastructural level, cells were surrounded by proteoglycan-rich matrices resembling those observed in situ. Reverse transcriptase PCR for collagen types I, II and X, and histological observations indicated the preservation of the chondrocytic phenotype for most cells in all types of matrices.

After 14 days of culture, both matrices were surfaced with a cartilaginous-like layer. Clusters of chondrocytes were present in type II-based matrices. No differences were observed with respect to chondrocyte behavior in type II matrices, with or without attached CS. These results thus show that crosslinked type II collagen matrices provides an appropriate environment for cartilage engineering.

According to a second embodiment of the invention, the type II collagen fibrils are mechanically comminuted to obtain fibril parts. In order to achieve this, use is made of cutting, for example in a Fritch Pulverisette 19.

This embodiment is represented by a method for the preparation of a matrix of mechanically modified type II collagen, comprising the steps of:
a) providing a source of type II collagen;
b) treating the source of type II collagen under high cutting to obtain mechanically modified type II collagen;
c) preparing a non-crosslinked type II collagen matrix thereof; and
d) crosslinking the matrix thus obtained.

This method is further illustrated in Example 3.

The source of type II collagen for both methods is preferably a source of cartilage, such as bovine tracheal cartilage.

The non-crosslinked type II collagen matrix is preferably prepared by lyophilization of an acidic dispersion of the modified type II collagen and the crosslinking of the matrix is performed using 1-ethyl-3-(3-dimethyl aminopropyl)carbodiimide (EDC) and N-hydroxysuccinimide (NHS).

According to another aspect of the invention it was found that also combinations of type I and II collagen matrices may be used in cartilage regeneration. As is shown in Example 2, crosslinked type I and type II collagen matrices, with and without attached chondroitin sulfate (CS), were implanted into full-thickness defects in the femoral trochlea of adolescent rabbits. The tissue response was evaluated 4 and 12 weeks after implantation by general histology, and two semi-quantitative histological grading systems.

Four weeks after implantation, type I collagenous matrices were completely filled with cartilage-like tissue. By contrast, type II collagenous matrices revealed predominantly cartilaginous tissue only at the superficial zone and at the interface of the matrix with the subchondral bone, leaving large areas of the matrix devoid of tissue. Attachment of CS promoted cellular ingrowth and cartilaginous tissue formation in both types of collagen matrices. Twelve weeks after implantation, the differences between the matrices were less pronounced. The subchondral defects were largely replaced by bone with a concomitant degradation of the matrices. The original cartilage contours in defects with type I collagen-based matrices were repaired with fibro-cartilaginous tissue. Defects containing type II matrices showed an increase in the amount of superficial cartilage-like tissue. From this it follows that different types of collagen matrices induce different tissue responses in full-thickness articular cartilage defects. Type I collagen-based matrices are superior to guide progenitor cells from a subchondral origin into the defect. In type II collagen-based matrices cell migration is less, but chondrocyte phenotype in the superficial zone is better maintained.

In view of this, the present invention thus also provides a composite matrix consisting of one or more first layers or parts of type I collagen, and one or more second layers or parts of type II collagen.

The matrices and composite matrices of the invention may further comprise one or more glycosaminoglycans (GAGs). In the human body GAGs are used as a depot for biological effector molecules. According to the invention the matrices can be either loaded with GAGs or with biological effector molecules or with both. The GAGs are preferably covalently coupled to the collagen in the matrix.

The glycosaminoglycans are preferably selected from the group consisting of chondroitin sulfate (CS), sulfated heparin, heparan sulfate, hyaluronate, keratan sulfate, dermatan sulfate.

The biological effector molecule can be selected from the group consisting of growth factors, enzymes, enzyme inhibitors, cytokines. Examples of such biological effector molecule are bone morphogenic proteins (BMPs), fibroblast growth factors (FGFs), transforming growth factor β (TGF-β).

All these matrices of the invention can be used in the treatment of conditions in which cartilage regeneration is required, such as arthritis, arthrose, and traumatic and congenital disease.

Although a preferred form of type II collagen substrate is a matrix as described herein, the substrate may also take on other forms such as films. To prepare films, type II collagen suspensions or solutions may be dried at a temperature of for example 22°C.

The invention is further illustrated in the examples that follow and that are not intended to limit the invention. In the Examples reference is made to the following figures:
**Fig. 1.** Sodium dodecyl sulphate-polyacrylamide gel electrophoresis. **(A)** bovine tracheal cartilage; **(B)** isolated type II atelocollagen.
**Fig. 2.** Immunostaining of type II collagen preparations for chondroitin sulfate. **(A)** native type II collagen; **(B)** type II atelocollagen; Bar indicates 50 µm.
**Fig. 3.** Scanning electron micrographs of air-sides of lyophilized collagen matrices. **(A)** type I collagen matrix; **(B)** type II atelocollagen matrix; Bar indicates 100 µm.
**Fig. 4.** Transmission electron micrographs of crosslinked collagen matrices. **(A)** type I collagen matrix; arrowheads: collagen fibrils; **(B)** type II atelocollagen matrix. Ultrathin sections (70 nm) were poststained with lead citrate and uranyl acetate. Bar indicates 1 µm; mf: matrix fibril.
**Fig. 5.** Immunostaining of crosslinked type II atelocollagen-chondroitin sulfate matrix for chondroitin sulfate. Bar indicates 50 µm.
**Fig. 6.** DNA content in crosslinked collagenous matrices seeded with chondrocytes as a function of culture time. Values are mean ± SD (n=5). COL(II): type II atelocollagen matrix; COL(II)-CS: type II atelocollagen-chondroitin sulfate matrix; COL(I)-CS: type I collagen-chondroitin sulfate matrix
**Fig. 7.** GAG content in crosslinked collagenous matrices seeded with chondrocytes as a function of culture time. Values are mean ± SD (n=5), and corrected for the attached CS content of matrices.
**Fig. 8.** Light microscopy of chondrocytes cultured on crosslinked collagenous matrices. **(A)** type I collagen-chondroitin sulfate matrix, 7 days after cell-seeding; **(B)** type II atelocollagen-chondroitin sulfate matrix, 7 days after cell-seeding; **(C)** type I collagen-chondroitin sulfate matrix, 14 days after cell-seeding; **(D)** type II atelocollagen-chondroitin sulfate matrix, 14 days after cell-seeding; **(E)** type II atelocollagen-chondroitin sulfate matrix, 14 days after cell-seeding; arrow indicates chondrocyte cluster; H&E staining. Bar indicates 50µm.
**Fig. 9.** Scanning electron micrographs of chondrocytes on a crosslinked type II atelocollagen-chondroitin sulfate matrix, 7 days after cell-seeding. **(A)** arrows indicate chondrocytes, bar indicates 100 µm; **(B)** bar indicates 50 µm.
**Fig. 10.** Transmission electron micrographs of proteoglycans (arrow heads). **(A)** pericellular matrix of chondrocyte in bovine tracheal cartilage; **(B)** crosslinked type II atelocollagen matrix, 14 days after cell-seeding. Samples were fixed in the presence of cupromeronic blue. Ultrathin sections (70 nm) were not poststained. Bar indicates 500 nm; ch: chondrocyte; mf: matrix fibril.

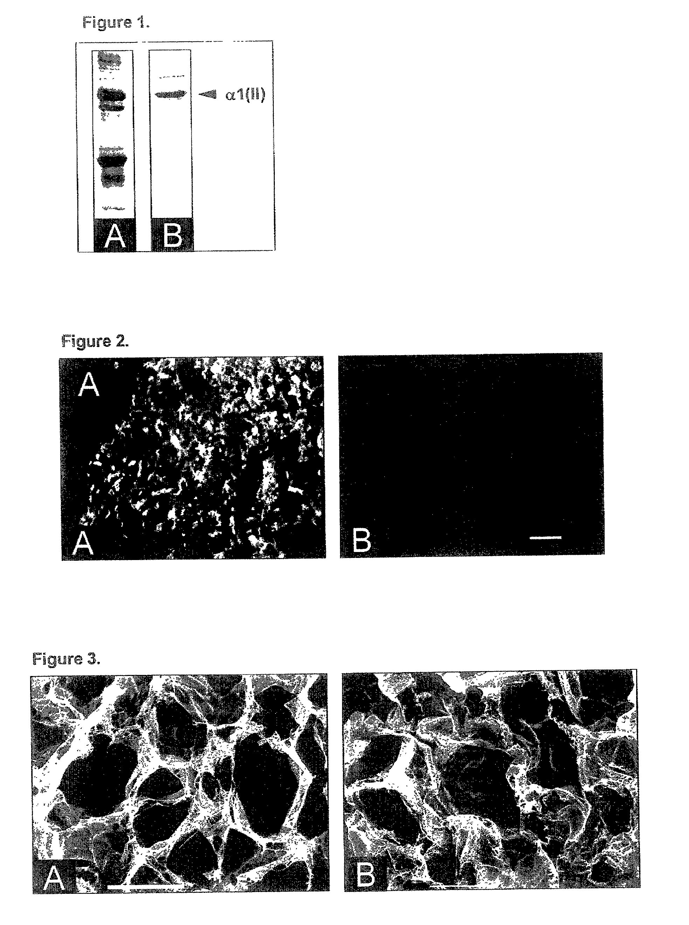

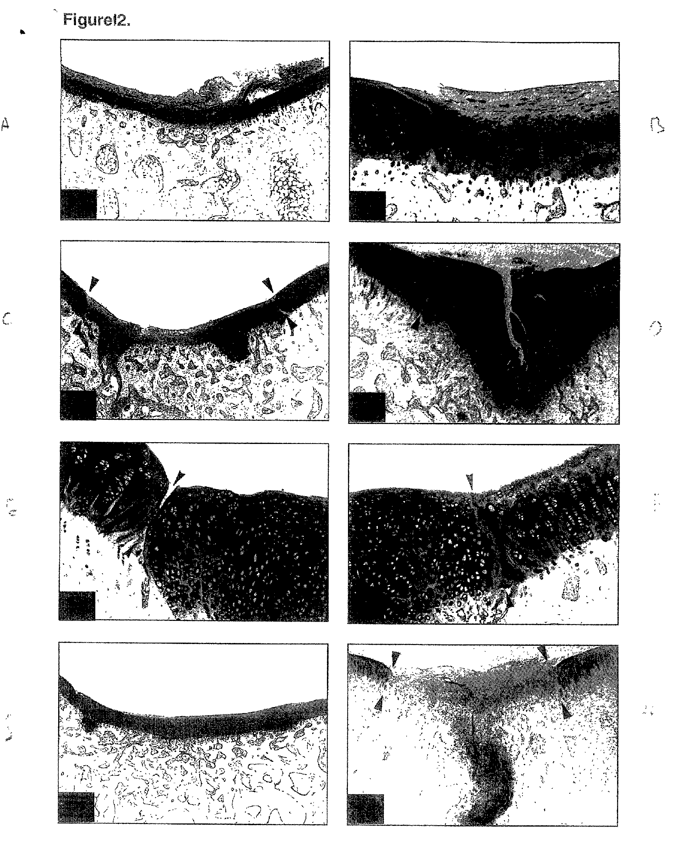

**Fig 11.** Light microscopical evaluation of full-thickness articular cartilage defects, with and without collagenous matrices, 4 weeks after implantation (toluidine blue staining). **(A)** Crosslinked collagen type I matrix; arrowheads indicate transition between newly formed tissue and adjacent host tissue (25x) **(B)** Crosslinked collagen type I matrix; arrowheads indicate collagen matrix structure (200x). **(C)** Crosslinked collagen type II matrix; arrowheads indicate transition between newly formed tissue and adjacent host tissue; asterix indicates empty matrix (25x). **(D)** Crosslinked collagen type II matrix. Enlargement of the rectangle in Figure 1C; asterix indicates empty matrix (100x). **(E)** Control defect, arrowheads indicate transition between newly formed tissue and adjacent host tissue.
**Fig 12.** Light microscopical evaluation of full-thickness articular cartilage defects, with and without collagenous matrices, 12 weeks after implantation (toluidine blue staining). **(A)** Crosslinked collagen type I matrix (25x). **(B)** Crosslinked collagen type I matrix. Transition zone of pre-existing cartilage and the newly formed surface (100x). **(C)** Crosslinked type II collagen matrix; arrowheads indicate transition between newly formed tissue and adjacent host tissue (25x). **(D)** Crosslinked type II collagen-CS matrix; arrowhead indicates transition between newly formed tissue and adjacent host tissue (25x). **(E)** Crosslinked type II collagen-CS matrix; arrowhead indicates transition between newly formed tissue and adjacent host tissue (100x). **(F)** Crosslinked type II collagen-CS matrix; arrowhead indicates transition between newly formed tissue and adjacent host tissue (100x). **(G)** Control defect; arrowheads indicate transition between newly formed tissue and adjacent host tissue (25x). **(H)** Control defect (25x).

### EXAMPLES

### EXAMPLE 1

### Preparation of type II collagen matrix and use thereof for culturing chondrocytes

### INTRODUCTION

The potential of insoluble type I and II matrices for cartilage engineering was studied in vitro by culturing chondrocytes. Cell-seeded matrices were assessed morphologically by light and electron microscopy, and biochemically by DNA and GAG analyses. In addition, in order to obtain insight into the differentiation status of the chondrocytes, reverse transcriptase PCR was performed for collagen types I, II, and X.

### MATERIALS AND METHODS

### Collagen isolation and matrix preparation

Native insoluble type I collagen was isolated from bovine achilles tendon as described [Pieper JS et al., Biomaterials 1999:20:847-858.]. Type II collagen was isolated from bovine tracheal cartilage. Initially it was tried to prepare native insoluble type II collagen applying the same methodology as used for type I collagen. This, however, was unsuccessful since even after prolonged washing procedures, CS remained associated with type II collagen. In addition, native 10 type II collagen did not properly swell in acetic acid which is necessary for the preparation of matrices. Therefore, collagen was treated with pepsin to prepare type II atelocollagen (collagen without telopeptides), which can be purified.

The final procedure used was as follows. Trachea was defatted, stripped from surrounding conjunctiva, cut into small pieces (0.5 cm³), and incubated with demineralized water (3 times x 0.5 h). All procedures were performed at 4°C. Subsequently, the cartilage was washed in 8 vol of 50 mM Tris-HCl (pH 7.2), containing 25 mM EDTA-Na₂, and 2 mM N-ethylmaleimide (NEM) for 16 h, followed by 4 washings in 8 vol of the same buffer containing 4 M guanidine-HCl for 24 h. After incubation in 0.5 M HAc (pH 2.5) (8 vol, 4 times x 24 h), the collagen was digested by incubation in 8 vol 0.5 M HAc (pH 2.5) containing 8.10⁶ units pepsin/liter (Sigma Chemical Co., P-6887) for 16 h. After centrifugation (20,000 x g, 20 min), NaCl was added to the supernatant to a final concentration of 0.86 M, and the collagen was allowed to precipitate for 16 h. Collagen was centrifuged (20,000 x g, 20 min), and dissolved in 0.5 M HAc (pH 2.5) (8 vol, 16 h). Precipitation and solubilization was repeated three times. Subsequently, the atelocollagen was aggregated by dialysis against 0.02 M phosphate buffer (pH 7.4).

After centrifugation (20,000 x g, 20 min), the collagen was washed in distilled water (2 times for 1 h), and centrifuged again (20.000 x g, 20 min). Finally, the collagen was lyophilized.

Purity of the type II atelocollagen preparations was evaluated using SDS-PAGE, amino acid analysis, and immunofluorescence [Pieper et al., 1999, supra]. Porous collagen matrices were obtained after lyophilization of an acidic dispersion (0.8 % (w/v)) of type I or type II collagen as previously described [Pieper et al., 1999, supra]. Matrix morphology was analysed using scanning and transmission electron 11 microscopy [Pieper et al., 1999, supra; Pieper JS et al., Biomaterials 2000; 21:581-593].

### Isolation and purification of chondroitin sulfate

CS was isolated and purified from bovine tracheal cartilage using extensive papain digestion, mild alkaline borohydride treatment, and DEAE ion exchange chromatography, as described [Pieper et al., 2000, supra].

### Crosslinking of collagen matrices

Crosslinking of collagenous matrices, in the presence and absence of CS, was performed using 1-ethyl-3-(3-dimethyl aminopropyl)carbodiimide (EDC) (Fluka Chemie, Buchs, Switzerland) and N-hydroxysuccinimide (NHS) [Pieper et al., 1999, supra; Pieper et al., 2000, supra]. Briefly, collagen matrices of 50 mg dry weight were incubated for 0.5 h at 20°C in 20 ml 40 % (v/v) ethanol containing 50 mM 2-morpholinoethane sulfonic acid (MES) (Fluka Chemie) (pH 5.0). Subsequently, matrices were crosslinked by immersion for 4 h at 20°C in 15 ml 40 % (v/v) ethanol containing 50 mM MES (pH 5.0), 33 mM EDC, 20 mM NHS, with or without 2.75 % (w/v) CS.

### Characterization of collagen matrices

Denaturation temperature (Td): The Td, indicative for the degree of matrix crosslinking, was measured using differential scanning calorimetry [Pieper et al., 1999, supra].

Amine group content: The amine group content of the matrices was monitored spectrophotometrically using 2,4,6-trinitrobenzenesulfonic acid [Pieper et al., 1999, supra].

Immobilized CS content: The amount of covalently attached CS was determined by hexosamine analysis using p-dimethylaminobenzaldehyde after acid hydrolysis [Pieper et al., 1999, supra].

Matrix degradation by collagenase: Matrices of about 5 mg dry weight were incubated for 0.5 h in 1 ml 0.1 M Tris-HCl (pH 7.4), containing 50 mM CaCl₂ at 37°C. Subsequently, 200 U bacterial collagenase (Clostridium histolyticum, EC 3.4.24.3, Sigma Chemical Co.) in 1 ml 0.1 M Tris-HCl (pH 7.4) was added. After incubation for 4.5 h at 37°C, reaction was stopped by the addition of 0.2 ml 0.25 M EDTA and cooling on ice. After centrifugation (5,000 g, 15 min, 4°C), precipitates were washed in distilled water containing 10 mM EDTA (3 times x 0.5 h, 4°C), in distilled water (3 times x 0.5 h, 20°C) and lyophilized. Matrix degradation was determined from the weight of residual matrix, and expressed as a percentage of the original weight.

### Isolation of chondrocytes

Bovine articular chondrocytes were isolated from metaphalangeal joints of adolescent cows. Cartilage slices were aseptically collected in RPMI 1640 medium (Flow Laboratories, Irvine, UK) containing collagenase B (Sigma Chemical Co.), 0.1 % penicillin, and 0.1 % streptomycin. After incubation for 16 h at 37°C, matrix debris was removed by sedimentation and the supernatant centrifuged (1000 x g, 10 min). The cell pellet was resuspended in 0.9 % sterile saline solution, and centrifuged again (1000 x g, 10 min). Finally, the pellet was resuspended in culture medium (RPMI 1640 containing 10 % fetal calf serum, 1 % pyruvate, 0.1 % penicillin, and 0.1 % streptomycin), centrifuged (1000 x g, 10 min), and resuspended again in the same medium.

### Culturing of chondrocytes

Crosslinked collagenous matrices were washed with 70 % (v/v) ethanol (4 times x 30 min, 20°C), followed by washing with sterile 0.9 % saline (5 times x 15 min, 20°C). Matrices, 6 mm in diameter and approximately 1.5 mm thick, were placed in 96-wells plates with the porous air-side (surface in contact with air during lyophilization) upwards to facilitate cellular penetration. After seeding the matrices with 200 µl cell suspension containing 7 x 10⁵ chondrocytes, the 96- wells plates were gently centrifuged (1000 x g, 3 min). Chondrocytes were cultured at 37°C, 5 % CO₂ and 95 % humidity for time periods up to 14 days. After day 1 and day 7, cell-seeded matrices were transferred to new 96-wells plates in order to eliminate cells attached to the culture plate. Culture medium was changed daily. Cultures were terminated at day 1, 3, 7, and 14.

### DNA and glycosaminoglycan (GAG) content

The DNA content, a measure for cell proliferation, was evaluated using the Hoechst 33258 fluorescent dye assay [Kim YJ et al., Anal Biochem 1988; 174:168-176]. Cell-seeded matrices were digested in 250 µl 0.2 M NaCl, 0.1 M NaAc, 0.01 M L-cysteine-HCl, 0.05 M EDTA-NA₂ (pH 6.0) containing 16 U papain (Sigma Chemical Co., p-3125) for 16 h at 65°C. To 100 µl papain digest, 100 µl 0.02 M Tris-HCl (pH 8.0) containing 0.1 % sodium dodecyl sulphate was added. The mixture was incubated for 30 min at 60°C. A 100 µl aliquot was analysed for total DNA by the addition of 1 ml Hoechst solution (0.2 µg Hoechst 33258/ml) (Riedel-De Haen AG, Hannover, Germany). The fluorescence of the samples was measured at an excitation of 365 nm and an emission of 458 nm. Calf thymus DNA (Boehringer, Mannheim, Germany) was used as a standard.

The GAG content in cell-seeded matrices was evaluated using a modified Farndale assay [Van de Lest CHA et al., Biochim Biophys Acta 1994; 1201:305-311]. Aliquots of 25 µl of the papain digest were assayed for GAG after the addition of 1.2 ml 1,9-dimethyl methylene blue. The absorbance was measured at 535 nm. CS was used as a standard.

### Light microscopy

Cell-seeded matrices (n=3) from day 7 and 14 of culture were fixed in 0.1 M phosphate buffer (pH 7.4) containing 2 % (v/v) glutaraldehyde for at least 24 h at 4°C. Matrices were embedded in paraffin, and sections (5 µm) were stained with hematoxylin and eosin, and with alcian blue.

### Scanning electron microscopy (SEM)

Samples were prepared for SEM as previously described [Pieper et al., 1999, supra]. Briefly, cell-seeded matrices (n=2) were fixed in 0.1 M phosphate buffer (pH 7.4) containing 2 % (v/v) glutaraldehyde for at least 24 h at 4°C. Matrices were critical point dried, sputtered with gold, and studied using a JEOL JSM-6310 SEM apparatus.

### Transmission electron microscopy (TEM)

To visualize GAGs, matrices were processed according to the critical electrolyte concentration method using cupromeronic blue (CB) (Seikagaku Co., Tokyo, Japan) [Pieper et al., 2000, supra]. CB specifically stains GAGs. Briefly, matrices were fixed and stained in 25 mM sodium acetate buffer (pH 5.6) containing 2.5 % (w/v) glutaraldehyde, 0.2 % (w/v) CB, and 0.2 M MgCl₂. Embedding was in Epon. Ultrathin sections (70 nm) were poststained with lead citrate and uranyl acetate, and examined in a JEOL electron microscope at 70 kV.

### Reverse transcriptase (RT)-PCR

To obtain information about the differentiation status of chondrocytes in the cell-seeded matrices, mRNA levels of type I, type II, and type X collagen were assessed using RT-PCR. RNA from cell-seeded matrices was isolated using the RNeasy kit (Qiagen GmbH, Germany), treated with DNase (Life Technologies), and transcribed into cDNA with moloney-murine leukemia virus reverse transcriptase (Life Technologies), and oligo(dT)15 primers (Eurogentic, Liege, Belgium). cDNA was amplified using Taq DNA polymerase (Life Technologies). To estimate the relative mRNA levels, samples of 5 µl were taken at increasing cycle numbers with a 2 cycle interval, representing approximately a four-fold increase in mRNA. The PCR products were electrophoresed in 1.6 % agarose gels containing ethidium bromide. The cycle number at which the products were first detected was used as a semi-quantitative measure for the amount of specific mRNA present in the isolated RNA. Glyceraldehyde 3-phosphate dehydrogenase (GAPDH) mRNA levels were taken as internal controls.

### RESULTS

### Type II collagen isolation and matrix preparation

Type II atelocollagen was isolated and purified from bovine tracheal cartilage. SDS-PAGE shows a profile typical for type II collagen, and indicates that the preparation is essentially free from contaminating proteins (Fig. 1). The amino acid composition is also indicative for collagen, and reveals an increase in glycine and hydroxyproline residues, relative to tracheal cartilage (Table 1).

**Table 1.**

| Amino acid composition of bovine tracheal cartilage and type II collagen | | |
|---|---|---|
| Amino acid residue | Tracheal cartilage | Type II collagen |
| Arginine | 53 ± 1 | 53 ± 2 |
| Hydroxyproline | 42 ± 1 | 87 ± 1 |
| Serine | 38 ± 2 | 24 |
| Asparagine/aspartic acid | 71 ± 1 | 47 |
| Glutamine/glutamic acid | 102 ± 2 | 85 ± 1 |
| Threonine | 42 ± 2 | 20 |
| Glycine | 213 ± 4 | 321 ± 3 |
| Alanine | 84 ± 1 | 104 ± 1 |
| Proline | 109 ± 6 | 120 ± 2 |
| Methionine | 11 | 8 |
| Valine | 49 ± 2 | 19 ± 1 |
| Phenylalanine | 24 ± 1 | 13 ± 1 |
| Isoleucine | 29 ± 1 | 14 ± 1 |
| Leucine | 52 | 27 ± 2 |
| Hydroxylysine | 23 ± 2 | 28 |
| Histidine | 14 ± 2 | 5 |
| Lysine | 31 ± 1 | 21 |
| Tyrosine | 13 ± 3 | 3 ± 1 |
| Values are mean ± SD(n=3), expressed per 1000 amino acid residues and not corrected for destruction during hydrolysis. | | |

Immunostaining of type II collagen preparations for CS is shown in Figure 2. Native insoluble type II collagen was prepared, applying the same methodology as used for type I collagen [Pieper et al., 1999, supra]. Although extensive and prolonged extraction procedures with 4 M guanidine-HCl were used for the purification of native type II collagen, residual cartilage PGs can still be observed (Fig. 2A). By contrast, the type II atelocollagen preparations are essentially free from residual PGs (Fig. 2B), which favors these preparations for the development of biocompatible type II collagen matrices.

Collagenous matrices were prepared by lyophilization. Both type I and type II collagen matrices reveal a similar morphology. The air-sides of both matrices (surface in contact with air during lyophilization) contain porous structures with pore-diameters ranging from 50 to 100 µm (Fig. 3). Inner matrix structures are comprised of lamellae which form a highly porous interconnecting network of channels.

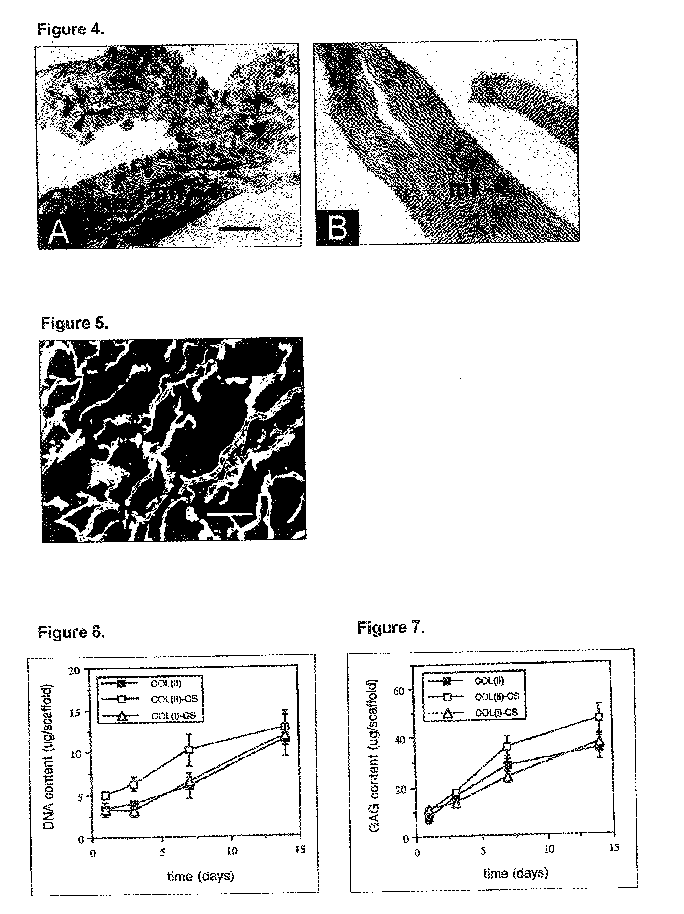

At the ultrastructural level, matrix structures of type I collagen matrices show the presence of native fibrils with the characteristic D-banding periodicity (Fig. 4A). By contrast, the type II atelocollagen matrices reveal filamentous matrix structures rather than fibrils, which is in line with their monomeric nature (Fig. 4B).

### Matrix characteristics

The physico-chemical characteristics of various collagenous matrices are presented in Table 2.

**Table 2.**

| Physico-chemical characteristics of various collagen matrices. | | | | |
|---|---|---|---|---|
| Matrix¹ | Td²(°C) | Amine groups³ (n/1000) | CS⁴ (mg/g matrix) | Degradation by⁵ collagenase (%) |
| COL(I)-NX | 61.3 ± 0.8 | 33 ± 3 | 133 ± 7 | 100 |
| COL(I)-CS | 75.1 ± 0.4 | 21 ± 2 | | 18 ± 4 |
| COL(II)-NX | 53.0 ± 1.5 | 31 ± 2 | | 100 |
| COL(II)-X | 73.9 ± 1.1 | 16 ± 1 | 180 ± 13 | 41 ± 6 |
| COL(II)-CS | 71.3 ± 0.9 | 18 ± 2 | | 72 ± 10 |

| | | | | |
|---|---|---|---|---|
| ¹ COL(I)-NX:non-crosslinked type I collagen matrix; COL(I)-CS:crosslinked type I collagen-chondroitin sulfate matrix; COL(II)-NX:non-crosslinked type II atelocollagen matrix; COL(II)-X:crosslinked type II atelocollagen matrix; COL(II)-CS:crosslinked type II atelocollagen-chondroitin sulfate matrix; | | | | |
| ² Denaturation temperature, values are mean ± SD (n=3); | | | | |
| ³ Expressed as amine group content per 1000 amino acids, values are mean ± SD (n=6); | | | | |
| ⁴ Values are mean ± SD (n=5). | | | | |
| ⁵ Expressed as percentage of degraded matrix, values are mean ± SD (n=4) | | | | |

Non-crosslinked type I collagen matrices have a Td of about 61°C. Non-crosslinked type II atelocollagen matrices have a lower Td of 53°C. This is probably due to the absence of native collagen fibrils, and hence a reduced amount of native crosslinks in these matrices. EDC/NHS crosslinking increases the Td and decreases the amine group content of both collagenous matrices. Additionally, resistance towards in vitro degradation by collagenase is increased. Crosslinked type II matrices are more susceptible towards degradation compared to crosslinked type I matrices. EDC/NHS crosslinking of type II matrices, in the presence of CS, results in a higher amount of attached CS, relative to type I matrices. Incorporated CS is distributed throughout the matrix (Fig. 5), and bioavailable, as demonstrated by its susceptibility towards digestion with glycosidases (data not shown).

### Matrices seeded with chondrocytes

### 1. DNA and GAG content in the matrices

Proliferation of and matrix production by chondrocytes were assessed by determination of the DNA and GAG content, respectively. All crosslinked matrices show a similar increase in the DNA content from approximately 3 µg DNA/matrix at day 1, to 12 µg DNA/matrix at day 14, indicating proliferation of chondrocytes (Fig. 6). Matrices also show a comparable increase in the total amount of retained GAGs from about 10 µg GAG/matrix to 40 µg GAG/matrix (Fig. 7), indicating synthesis of PGs.

### 2. Histology

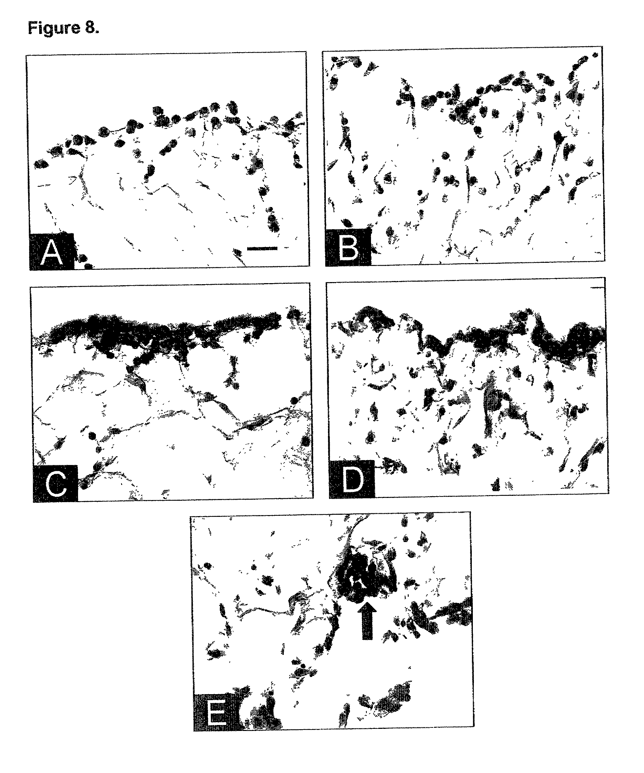

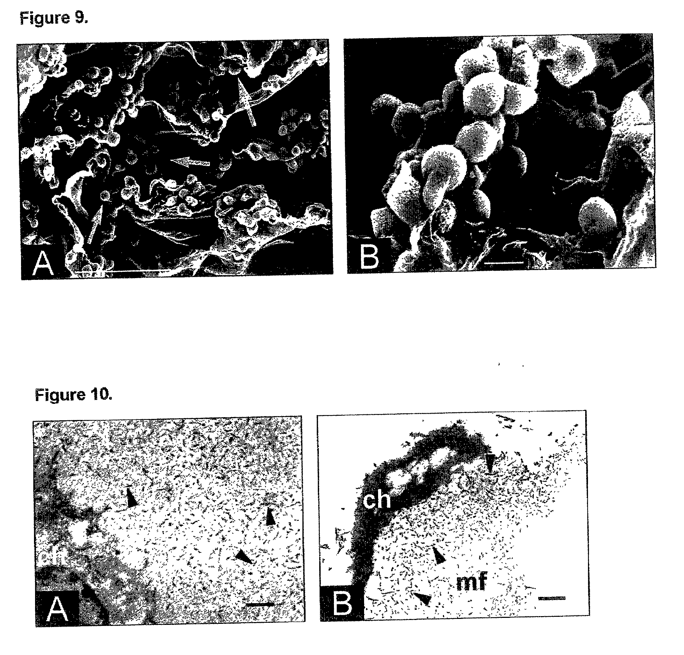

All EDC/NHS-crosslinked matrices maintain their structural integrity during culture. After 7 days of culture, cells at the margins of type I and type II collagen-CS matrices show a round morphology (Fig. 8A,B). This is confirmed with SEM (Fig. 9). The spherically shaped morphology is indicative for the chondrocytic phenotype. Type II collagen matrices show a distribution of chondrocytes in deeper parts, in contrast to type I collagen matrices.

After 14 days of culture, all matrices are surfaced with a cartilaginous-like layer. This layer is more dense at the periphery of type I collagen-CS matrices (compare Fig. 8C and D). In contrast to type I matrices, clusters of chondrocytes are again present inside type II matrices (Fig. 8E). Pericellular metachromatical staining with alcian blue in all matrices around cell-dense areas indicates the presence of PGs (data not shown).

The majority of the cells retain a round morphology during culture. The number of elongated cells, however, increases in all matrices from day 7 to 14. They are predominantly associated with collagen matrix structures. This suggests partial dedifferentiation of chondrocytes into a fibroblastic-like phenotype. At the histological level, no differences are observed between type II collagen matrices, with and without attached CS.

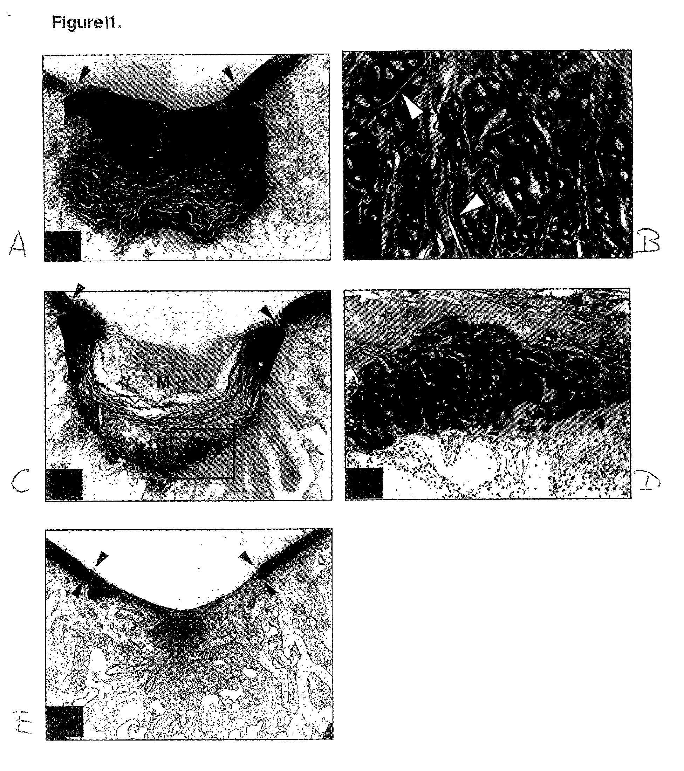

Preparations were also studied at the ultrastructural level using cupromeronic blue. This reagent specifically stains GAGs which appear as electron dense filaments. Cells are surrounded by PG-rich matrices resembling those observed in situ (compare Fig. 10A and B).

### 3. RT-PCR

RT-PCR was performed for the collagen types I, II and X to get an impression about the phenotype of the cells in the matrices during culture (Table 3).

**Table 3.**

| Reverse transcriptase PCR profile of mRNA for collagen types from chondrocytes cultured in various collagenous matrices. | | | | | | |
|---|---|---|---|---|---|---|
| Type collagen¹ | COL(II)-X | | COL(II)-CS | | COL(I)-CS | |
| | 1d | 14d | 1d | 14d | 1d | 14d |
| I | -8² | -7 | -7 | -6 | -7 | -6 |
| II | +2 | +3 | +2 | +1 | +2 | +1 |
| X | -14 | -15 | -12 | -13 | -14 | -15 |

| | | | | | | |
|---|---|---|---|---|---|---|
| COL(II)-X:crosslinked type II atelocollagen matrix; | | | | | | |
| COL(II)-CS:crosslinked type II atelocollagen-chondroitin sulfate matrix; | | | | | | |
| COL(I)-CS:crosslinked type I collagen-chondroitin sulfate matrix; ¹m RNA levels for the collagen types are expressed as the number of cycles from their respective GAPDH mRNA levels, and evaluated at day 1 (1d) and 14 (14d). ²-8 indicates 8 cycli later than GAPDH. | | | | | | |

At day 1 and 14, mRNA levels for type II collagen predominate in all matrices investigated, indicating preservation of the chondrocytic phenotype for most cells.

### DISCUSSION

Tissue-engineered cartilage constructs may provide functional analogs for the regeneration of articular cartilage defects. The biochemical composition of such a construct determines to a major extent cell behavior. In this in vitro study, type II collagen and CS, the main constituents of native articular cartilage, were used to prepare insoluble matrices. These matrices were analysed for sustaining chondrocyte growth and differentiation.

Porous type II collagen matrices were fabricated using highly purified type II atelocollagen preparations. EDC/NHS treatment of these matrices increased the denaturation temperature, and decreased the amine group content, demonstrating that crosslinking occurred. Crosslinking of type II collagen, in the presence of CS, resulted in a distribution of CS throughout the matrix. Incorporated CS remained bioavailable, as demonstrated by its susceptibility to glycosidases.

All EDC/NHS-crosslinked collagenous matrices had a similar porous morphology. They maintained their structural integrity during culture, and contained sufficient strength to be handled with forceps. This favors insoluble matrices for tissue engineering relative to hydrogels. Type II-based matrices showed a distribution of cells throughout the matrix, which facilitates an appropriate cartilage regeneration.

Bioactivity of cells cultured in the matrices was established by the increase of DNA and GAG. Cells in type I and type II collagen matrices were surrounded by PG-rich matrices resembling those observed in situ. The ultrastructural morphology of the PGs appeared similar to aggrecan.

RT-PCR for various types of collagen, and morphological observations indicated the preservation of the chondrocytic phenotype for most cells in all collagenous matrices. Preservation of the chondrocytic phenotype is a prerequisite for the generation of a cartilage-specific environment. If chondrocytes are grown in monolayer culture they dedifferentiate, obtain a fibroblast-like morphology and synthesize type I rather than type II collagen. The three-dimensional structure of the invention favors the formation of type II collagen.

After 14 days of culture, the type II matrices supported the formation of clusters of chondrocytes inside the scaffold, in contrast to type I collagen. These cellular nodules resemble juvenile repair attempts to synthesize new extracellular matrix.

These results show that crosslinked type II collagen matrices create an appropriate environment for 22 culturing chondrocytes and the generation of an engineered cartilage construct. Implantation of these matrices in full-thickness articular cartilage defects as shown in the following example further shows the value of type II collagen matrices.

### EXAMPLE 2

### Use of the type II collagen matrix of the invention for cartilage regeneration in vivo

### INTRODUCTION

In this Example, crosslinked type I and type II collagen matrices, with and without attached chondroitin sulfate (CS), were implanted into full-thickness articular cartilage defects in the trochlea of rabbits. Cartilage and subchondral bone remodeling was evaluated, 4 and 12 weeks after implantation, using histology, and two semi-quantitative histological grading systems.

Full-thickness defects involve both bone and cartilage. It will be shown here that composites of type I and type II collagen matrices may be used for the regeneration of such defects.

### MATERIALS AND METHODS

### 1. Preparation, crosslinking, and characterization of collagen matrices

Insoluble type I collagen was isolated from bovine achilles tendon using neutral salt and dilute acid extractions [Pieper et al., 1999; supra]. Type II collagen was isolated from bovine tracheal cartilage using pepsin digestion and specific salt precipitation [cf. Example 1]. CS was isolated from bovine tracheal cartilage using extensive papain digestion, and mild alkaline borohydride treatment [Pieper et al., 2000; supra].

Porous collagenous matrices were prepared by lyophilization of an acidic dispersion of the collagen preparations. Crosslinking of the collagen matrices, in the presence and absence of CS, was performed using 1-ethyl-3-(3-dimethyl aminopropyl) carbodiimide (EDC) (Fluka Chemie, Buchs, Switzerland) and N-hydroxysuccinimide (NHS) (Fluka Chemie) [Pieper et al., 1999, supra, Pieper et al., 2000, supra]. The degree of matrix crosslinking was analysed with respect to the denaturation temperature, amine group content, and the amount of covalently attached CS [Pieper et al., 1999, supra].

### 2. Animal study

Forty young male New Zealand white rabbits (mean weight 2570 ± 23 g, age 12 weeks) were used. Under general anaesthesia a medial incision was made over the right knee joint. After the joint was opened, the patella could be luxated with the leg in full extension. Two full-thickness defects (4 mm in diameter and 3 mm in depth) were created in the trochlea using a dental highspeed drill with saline irrigation. After implantation of matrices in the defects, the wound was carefully closed and the animal was allowed full weight bearing and normal daily activity in large rooms with a saw dust coverage on the floor. Empty defects were taken as controls. The rabbits were killed after 4 (20 rabbits, 4 rabbits per treatment) and 12 weeks (20 rabbits, 4 rabbits per treatment) by an overdose of Nembutal.

### 3. Histology

After killing of the animals, the knee was inspected macroscopically. The synovial tissue was evaluated for inflammatory reactions. Tissue blocks containing the defects and directly surrounding bone, were dissected with an oscillating saw under saline irrigation, and fixed in 4 % phosphate-buffered formalin (pH 7.4). After embedding in polymethylmethacrylate, sections (2 µm) were stained with hematoxylin and eosin, alcian blue, and toluidine blue. Representative sections through the center of the defect were scored in duplicate using a modification of the scoring system developed by O'Driscoll et al. [J Bone Joint Surg Am 1986;68:1017-1035] (Table 4).

**Table 4.**

| Histological scoring system for full thickness articular cartilage defects | |
|---|---|
| Characteristic | Score |
| **I Cellular morphology** | |
| Hyaline-like articular cartilage | 4 |
| Partial differential hyaline cartilage | 2 |
| Fibrous tissue | 0 |

| **II Surface characteristics** | |
|---|---|
| A. Regularity | |
| Smooth and intact | 3 |
| Horizontal fibrillation | 2 |
| Fissures to 25-100% of the depth of cartilage | 1 |
| Deep interruption of the surface many deep fibrillations | 0 |

| B. Thickness | |
|---|---|
| 100% of normal cartilage | 2 |
| 50-100% | 1 |
| 0-50% | 0 |

| C. Cellularity | |
|---|---|
| 75-100% of surface filled with cells | 3 |
| 50-75% | 2 |
| 25-50% | 1 |
| 0-25% | 0 |

| **III Chondrocyte clustering** | |
|---|---|
| None | 2 |
| < 25% | 1 |
| 25-100% | 0 |

| **IV Degenerative changes** | |
|---|---|
| Normal cellularity, no clustering, normal methachromatical | 3 |
| staining | |
| Normal cellularity, some cluster formation, moderate staining | 2 |
| Moderate hypocellularity, poor staining | 1 |
| Hypocellularity, no or very little staining | 0 |

| **V Restoration of the subchondral bone** | |
|---|---|
| Normal and straight | 4 |
| Slight contour changes | 2 |
| Large interruptions | 1 |
| Defect | 0 |

| **VI Integration** | |
|---|---|
| Both sides of repair tissue integrated with host cartilage | 2 |
| One side integrated | 1 |
| No integration | 0 |

Modified from O'Driscoll et al. (36)

A second scoring system was used to evaluate tissue ingrowth in the collagenous matrices 4 weeks after implantation (Table 5).

**Table 5**

| Histological scoring system for matrices in full-thickness articular cartilage defects | |
|---|---|
| Characteristic | Score |
| **Quality of tissue in matrix** | |
| Normal articular cartilage | 3 |
| Mostly cartilage tissue | 2 |
| Mostly fibrous tissue | 1 |
| Fibrous tissue | 0 |
| **Matrix filled with cells** | |
| 75-100% | 3 |
| 50-75% | 2 |
| 25-50% | 1 |
| 0-25% | 0 |

### RESULTS

### 1. Physico-chemical characteristics of matrices

The physico-chemical characteristics of the different collagenous matrices are presented in Table 6.

**Table 6**

| Physico-chemical characteristics of implanted collagen matrices | | | |
|---|---|---|---|
| Matrix^{a} | Td^{b}(°C) | Amine groups^{c} (n/100) | CS^{d} (mg/g matrix) |
| COL(I)-X | 76.5 ± 0.7 | 20 ± 2 | |
| COL(I)-CS | 75.1 ± 0.4 | 21 ± 2 | 133 ± 7 |
| COL(II)-X | 73.9 ± 1.1 | 16 ± 1 | |
| COL(II)-CS | 71.3 ± 0.9 | 18 ± 2 | 180 ± 13 |

| | | | |
|---|---|---|---|
| ^{a}COL(I)-X: crosslinked type I collagen matrix; | | | |
| COL(I)-CS: crosslinked type I collagen-chondroitin sulfate matrix; | | | |
| COL(II)-X: crosslinked type II collagen matrix; | | | |
| COL(II)-CS: crosslinked type II collagen-chondroitin sulfate matrix. ^{b}Denaturation temperature: values are mean ± SD (n=3). ^{c}Values are mean ± SD (n=6). ^{d}Values are mean ± SD (n=5). Non-crosslinked type I collagen has a Td of 61.3 ± 0.8°C and an amine group content of 33 ± 3 per 1000 amino acid residues. Non-crosslinked type II collagen has a Td of 53.0 ± 1.5°C and an amine group content of 31 ± 2 per 1000 amino acid residues. | | | |

EDC treatment of matrices increases the denaturation temperature, and decreases the amine group content, indicating that crosslinking occurred. The amount of CS attached to type I and type II collagen matrices is 13 % (w/w) and 18 % (w/w), respectively.

### 2. Clinical evaluation and gross morphology

No clear changes in walking pattern of the rabbits were observed. The animals moved freely through their housing. After killing and opening of the joint, no significant adhesions were found and the synovial tissue had a normal appearance without signs of inflammation. After opening of the joint cavity, no signs of inflammation were found either. The host cartilage around the defect sites showed macroscopically the white, light-red color of normal cartilage. At 4 weeks follow up, macroscopic inspection showed that all defects, including controls, were filled with material (see, however, point 3 below). The color of this material varied from white to transparent light-red, and occasionally resembled that of normal cartilage. No clear correlation was found between the treatment and the color of the implant site. At 12 weeks follow up, defects were generally filled up to the original contour of the trochlea. They had a more white appearance, and resembled more closely adjacent host cartilage.

### 3. Tissue response after 4 weeks

Two semi-quantitative histological grading systems were used for the evaluation of the tissue response. One grading system was used to evaluate the defect with respect to e.g. cell morphology, surface integrity, degenerative changes and subchondral bone remodeling (histological grading system Table 4). Another system was designed to evaluate cell ingrowth and morphology in the matrices (histological grading system Table 5). The mean histological scores of the various treatment modalities are presented in Table 7. Two types of tissue responses, can be distinguished, depending on the type of matrix used.

**Table 7**

| Mean histological scores for collagenous matrices implanted in full-thickness articular cartilage defects | | | | | | |
|---|---|---|---|---|---|---|
| Weeks after implantation | Scoring | COL(I)-X^{c} | COL-(I)-CS^{c} | COL(II)-X^{c} | COL(II)-CS^{c} | Control |
| 4 | defect^{a} | 13 ± 2 | 16 ± 1 | 8 ± 2 | 11 ± 6 | 12 ± 6 |
| 4 | matrix^{b} | 4 ± 1 | 5 ± 1 | 2 ± 1 | 3 ± 1 | |
| 12 | defect^{a} | 16 ± 1 | 12 ± 3 | 10 ± 5 | 11 ± 3 | 15 ± 1 |

| | | | | | | |
|---|---|---|---|---|---|---|
| ^{a}Scoring according to Table 4 (maximum score = 23); | | | | | | |
| ^{b}Scoring according to Table 5 (maximum score = 6); | | | | | | |
| ^{c}COL(I)-X: crosslinked type I collagen matrix; COL(I)-CS: crosslinked type I collagen-chondroitin sulfate matrix; COL(II)-X: crosslinked type II collagen matrix; COL(II)-CS: crosslinked type II collagen-chondroitin sulfate matrix; Control: empty defect. Values are mean ± SEM (n = 4) | | | | | | |

Type I collagen-based matrices are almost completely filled with cartilage-like tissue (Fig. 11A, Table 7). Cells are rounded, located in clear clusters, and surrounded by an intense metachromically-stained extracellular matrix (Fig. 11B). The surface of the defect is generally smooth, and follows the original contour of the trochlea. Demarcations between the cartilaginous tissue and the residual host cartilage are only occasionally observed.

Type II collagen-based matrices of the invention exhibit a more superficial tissue response (Fig. 11C). These matrices are only partially populated by cells, leaving large areas of the implant devoid of newly formed tissue (Fig. 11C,D). The tissue response consists of areas of fibrous-like tissue and cartilage-like tissue. The latter is found superficially and at the interface of the implant and the subchondral bone (Fig. 11D). The contours of the defect are not completely restored, leaving in most cases a central depression.

At 4 weeks, bone remodeling can be observed in the deep areas of the defects for all types of matrices. In case of cartilage-like tissue, chondral hypertrophy results in endochondral calcification. In some cases direct bone apposition is found on the matrix fibers.

Control defects show considerable subchondral tissue response (Fig. 11E). The subchondral bone is partially remodeled. The superficial area is mainly composed of fibrous or fibro-cartilaginous tissue, and generally in level with adjacent cartilage. Fissures or severe disruptions, however, are occasionally observed in the superficial central zone.

The histological scores (Table 7) also indicate that the tissue response in defects filled with type I collagen matrices is qualitatively better compared to defects filled with type II collagen matrices and control defects. In addition, irrespectively of the type of matrix, the attachment of CS appeared to enhance cellular ingrowth, and the amount of cartilage-like tissue.

### 4. Tissue response after 12 weeks

At 12 weeks after implantation, the differences between the matrices are less pronounced. In all defects, bone remodeling has resulted in an almost normal contour of the subchondral bone (Fig. 12A,C). The matrices are concomitantly degraded. Their structures are still partially visible in the superficial zone. Irrespective to the type of matrix, in some of the defects the repair tissue is slightly higher as compared to the host cartilage. Particularly in these cases there is a trend for some fibrillation and poor staining with alcian blue and toluidine blue in the superficial area of the defect (Fig. 12A,B,H). Poor staining of the adjacent host cartilage, and cluster formation of chondrocytes are only occasionally observed.

The superficial area of defects filled with type I collagen-based matrices is restored with fibro-cartilaginous tissue (Fig. 12A). This layer reveals congruence in thickness with the host cartilage. Horizontal fibrillation is occasionally found as is shown in Fig. 12A. Continuity between the repair tissue and the host is observed (Fig. 12B).

Type II collagen-based matrices show a clear increase in the amount of superficial cartilaginous tissue compared to week 4 (Fig. 12C,D). The original contour of the trochlea, however, still reveal a central depression or fissure (Fig. 12D). The cartilage-like tissue in the implant site is in most cases directly connected with the host cartilage (Fig. 12F). Occasionally, a fissure remains between the host and the repair tissue (Fig. 12E).

Control defects show continued bone remodeling, up to the normal level of the subchondral bone. In most cases the repair tissue is of a fibro-cartilaginous nature, the staining intensity with toluidine blue being clearly less compared to the adjacent host cartilage (Fig. 12G). In some cases, however, the controls reveal an appropriate restoration of the full-thickness defect, the newly formed superficial tissue being mainly of the chondrocytic phenotype and its thickness in level with the cartilage of the host (Fig. 12H). Some superficial horizontal fibrillation is observed. Occasionally, fissures are observed in the upper central parts of the newly formed tissue (not shown).

### DISCUSSION

Partial-thickness defects in which damage is limited to the cartilage, and which do not extent into the subchondral bone, never repair spontaneously. This is due to the absence of an appropriate source of cells for repair, and indicates the value of the subchondral bone marrow as a pool for non-differentiated mesenchymal cells.

The implantation of collagenous matrices in full-thickness defects may guide the infiltration, proliferation, and differentiation of these progenitor cells, and improve the healing response. In this example, crosslinked type I and type II collagen matrices, with and without attached CS, were implanted into full-thickness articular cartilage defects in adolescent rabbits. Matrices were implanted without precultured cells, allowing the study of the contribution of the host cells to the healing response.

Two types of tissue responses were observed at 4 weeks after implantation, depending on the type of matrix implanted. Type I collagen-based matrices were completely filled with cartilage-like tissue. Since no blood vessels were found in the area of the original defect, cells probably populate the matrix without vascular invasion.

In this example, in contrast to type I matrices, type II collagenous matrices only revealed cartilaginous tissue at the superficial zone and at the interface of the matrix with the subchondral bone, leaving large areas of the matrix devoid of newly formed tissue.

Twelve weeks after implantation, the differences between the matrices were less pronounced. Bone remodeling resulted in an almost normal contour of the subchondral bone. Matrices were concomitantly degraded without inducing inflammatory or foreign body reactions, demonstrating their biocompatibility. Both types of matrices generally showed a good integration with the subchondral bone and adjacent cartilage. This is important since micromotion between repair and host tissue may initiate cartilage degeneration. The original cartilage contours in defects with type I collagen matrices were restored with fibro-cartilaginous tissue. Type II matrices favored the maintenance of the chondrocytic phenotype in the superficial zone.

The attachment of CS appeared to enhance the population by cells, and the amount of cartilage-like tissue in both types of matrices. Favorable effects of CS with respect to chondrocyte proliferation and proteoglycan retention were not observed, however, in example 1 using type II collagen matrices in vitro. The favorable effects of CS in this in vivo study may reside in the fact that its presence creates an environment for cells which more closely resembles the extracellular matrix of cartilage (about 25 % of the dry weight of cartilage is CS). In addition, the presence of glycosaminoglycans preserves porous matrix structures, which may facilitate cellular ingrowth.

The results of this example demonstrate that a composite matrix consisting of a deep layer of type I collagen for subchondral recruitment of progenitor cells, and of a more superficial layer of type II collagen for maintenance of the chondrocyte phenotype, is a preferred matrix for the regeneration of full-thickness articular cartilage defects.

### EXAMPLE 3

### Isolation of collagen type II in a Fritch Pulverisette 19

An alternative starting material for preparing the matrices of the invention are mechanically disrupted collagen type II fibrils.

Collagen type II was isolated and purified from bovine tracheal cartilage. Cartilage was defatted, stripped from surrounding conjunctiva and pulverized at -196°C by means of a Fritch Pulverisette 19. The sieve diameter was 0.5 mm. The preparation was washed with 8 vol of demineralized water (3 times 0.5 h, 4°C), followed by washing with 8 vol of 50 mM Tris -HCl (pH 7.2), containing 25 mM EDTA-Na₂, and 2 mM N-ethylmaleimide (NEM) for 16 h at 4°C, and by 4 washings in 8 vol of the same buffer containing 4 M guanidine-HCl for 24 h at 4°C. The preparation was incubated in 0.5 M HAc (pH 2.5) (8 vol, 2 times 24 h, 4°C). After centrifugation (20,000 x g, 30 min, 4°C) the pellet was suspended in 8 vol of 0.5 M HAc (pH 2.5) and incubated for 72 h at 4°C while stirring. After centrifugation (20,000 x g, 30 min, 4°C) the pellet was obtained consisting of 2 layers. The fluffy upper layer, containing purified collagen type II, was collected and washed twice with 8 vol 0.5 M HAc (pH 2.5) for 4 h at 4°C. Finally, the collagen was lyophilized. Purity of the type II collagen preparation was established using SDS-PAGE, amino acid analysis and immuno fluorescence.

### EXAMPLE 4

### Preparation of composite type I and II collagen matrices

Composite collagen matrices for use in the regeneration of full-thickness defects were prepared as follows.

Collagen type I suspensions were prepared by incubation of 1.0 g insoluble type I collagen in 100 ml HAc (pH 2.5) at 4°C for 16 h and homogenization at 4°C by means of a teflon-glass Potter-Elvejhem homogenizer with a intervening space of 0.35 mm, followed by deaeration under vacuum to remove entrapped air bubbles. Finally, the collagen was poured into polystyrene culture flasks, quickly frozen at -80°C and lyophilized, resulting in porous collagen matrices.

Insoluble type II collagen suspensions were prepared according to Example 3, poured onto the lyophilized collagen type I porous matrices and quickly frozen at -80°C and lyophilised, resulting in a porous composite matrix.

EDC crosslinking of composite type I and II collagen matrix in the absence and presence of chondroitin sulfate was performed using 1-ethyl-3-(3-dimethyl aminopropyl)carbodiimide (EDC) and N-hydroxysuccinimide (NHS). Collagen matrices of 50 mg dry weight were incubated for 30 min in 20 ml 50 mM 2-morpholinoethane sulphonic acid (MES) (pH 5.5) containing 40% (v/v) ethanol. Subsequently, the matrices were crosslinked by immersion in 19.5 ml 50 mM MES (pH 5.5) containing 33 mM EDC and 6 mM NHS, containing 40% (v/v) ethanol. After incubation for 4 h, The matrices were washed twice in 0.1 M Na₂HPO₄ (pH 9.1) for 1 h. Finally, the matrices were washed with 1M NaCl for 2 h and with 2 M NaCl for 1 day (with 6 changes of washing solution), followed by washing with distilled water and lyophilization.

EDC crosslinking of collagen was also performed, as described above, in the presence of 2.75% (w/v) chondroitin sulphate (CS).

## Claims

1. Matrix for use in engineering or regeneration of cartilage, which matrix consists of a network of modified type II collagen.

2. Matrix as claimed in claim 1, wherein the modified type II collagen network is crosslinked.

3. Matrix as claimed in claim 2, wherein the modified type II collagen is type II atelocollagen.

4. Matrix as claimed in claim 3, wherein the type II atelocollagen is obtainable by enzymatic degradation of type II collagen.

5. Matrix as claimed in claim 4, wherein the enzymatic degradation is performed with pepsin.

6. Matrix as claimed in claim 1 or 2, wherein the modified type II collagen comprises type II collagen fibrils that have been treated with high cutting to obtain fibril parts.

7. Matrix as claimed in claims 1-6, further comprising one or more glycosaminoglycans (GAGs).

8. Matrix as claimed in claim 7, wherein the glycosaminoglycans are selected from the group consisting of chondroitin sulfate (CS), sulfated heparin, heparan sulfate, hyaluronate, keratan sulfate, dermatan sulfate.

9. Matrix as claimed in claims 1-8, further comprising one or more biological effector molecules.

10. Matrix as claimed in claim 9, wherein the biological effector molecule is selected from the group consisting of growth factors, enzymes, enzyme inhibitors, cytokines.

11. Matrix as claimed in claim 9 or 10, wherein the biological effector molecule is selected from the group consisting of bone morphogenic proteins (BMPs), fibroblast growth factors (FGFs), transforming growth factor β (TGF-β).

12. Method for the preparation of a matrix as claimed in claims 1-5, comprising the steps of:
a) providing a source of type II collagen;
b) enzymatically digesting the source of type II collagen to obtain enzymatically modified type II collagen;
c) preparing an non-crosslinked type II collagen matrix thereof; and
d) crosslinking the matrix thus obtained.

13. Method for the preparation of a matrix as claimed in claims 6, comprising the steps of:
a) providing a source of type II collagen;
b) treating the source of type II collagen under high cutting to obtain mechanically modified type II collagen;
c) preparing an non-crosslinked type II collagen matrix thereof; and
d) optionally crosslinking the matrix thus obtained.

14. Method as claimed in claim 12 or 13, wherein the source of type II collagen is a source of cartilage.

15. Method as claimed in claim 12, wherein the enzymatic digestion of the type II collagen is performed with pepsin to obtain type II atelocollagen.

16. Method as claimed in claims 11-15, wherein the non-crosslinked type II collagen matrix is prepared by lyophilization of an acidic dispersion of the modified type II collagen.

17. Method as claimed in claims 12-16, wherein the crosslinking of the matrix is performed using 1-ethyl-3-(3-dimethyl aminopropyl)carbodiimide (EDC) and N-hydroxysuccinimide (NHS).

18. Method for the preparation of a matrix as claimed in claims 6-10, wherein the GAG's are covalently Linked to the type II collagen in the matrix.

19. Type II collagen matrix obtainable by any one of the methods as claimed in claims 12-18.

20. Matrix as claimed in claims 1-11 and 19 for use in the treatment of conditions in which cartilage regeneration is required.

21. Matrix as claimed in claim 20, wherein the condition in which cartilage regeneration is required is selected from the group consisting of arthritis, arthrose, traumatic and congenital diseases.

22. Composite matrix of one or more layers or parts of a matrix as claimed in claims 1-11 or 19 and one or more layers or parts of a type I collagen matrix.

23. Composite matrix as claimed in claims 22, further comprising one or more glycosaminoglycans (GAGs).

24. Composite matrix as claimed in claim 23, wherein the glycosaminoglycans are selected from the group consisting of chondroitin sulfate (CS), sulfated heparin, heparan sulfate, hyaluronate, keratan sulfate, dermatan sulfate.

25. Composite matrix as claimed in claims 23 or 24, further comprising one or more biological effector molecules.

26. Composite matrix as claimed in claim 25, wherein the biological effector molecule is selected from the group consisting of growth factors, enzymes, enzyme inhibitors, cytokines.

27. Composite matrix as claimed in claim 25 or 26, wherein the biological effector molecule is selected from the group consisting of bone morphogenic proteins (BMPs), fibroblast growth factors (FGFs), transforming growth factor β (TGF-β).

28. Composite matrix as claimed in claims 22-27 for use in the treatment of conditions in which cartilage regeneration is required.

29. Composite matrix as claimed in claim 23, wherein the condition in which cartilage regeneration is required is selected from the group consisting of arthritis, arthrose, traumatic and congenital diseases.
